# EUROPÄISCHE PATENTSCHRIFT

(11) **EP 3 731 785 B1**
(45) Veröffentlichungstag und Bekanntmachung des Hinweises auf die Patenterteilung: **11.10.2023**
(21) Anmeldenummer: 18804596.7
(22) Anmeldetag: 16.11.2018
(51) Int. Cl.: A61F 2/50

(54) **COMPUTERIMPLEMENTIERTES VERFAHREN UND SYSTEM ZUR HERSTELLUNG EINER ORTHOPÄDISCHEN VERSORGUNG**
COMPUTER IMPLEMENTED METHOD AND SYSTEM FOR MANUFACTURING AN ORTHOPAEDIC DEVICE
PROCÉDURE IMPLÉMENTÉ PAR ORDINATEUR ET SYSTÈME POUR LA FABRICATION D'UN DISPOSITIF ORTHOPÉDIQUE

(30) Priorität: 27.12.2017 DE 102017131323
(43) Veröffentlichungstag der Anmeldung: 04.11.2020
(73) Patentinhaber: Mecuris GmbH, 80337 München (DE)
(72) Erfinder: OPITZ, Manuel, 80469 München (DE); GUNDLACK, Felix, 91074 Herzogenaurach (DE); SCHNAUBELT, Max, 80686 München (DE); RIETH, Clemens, 71032 Böblingen (DE)
(74) Vertreter: Meissner Bolte Partnerschaft mbB
(86) Internationale Anmeldenummer: PCT/EP2018/081579
(87) Internationale Veröffentlichungsnummer: WO 2019/129419

(56) Entgegenhaltungen:
- US-A1- 2012 281 013
- US-A1- 2014 180 185
- US-A1- 2015 343 709
- US-A1- 2017 246 013
- US-A1- 2017 360 578

## Beschreibung

Die Erfindung betrifft ein computerimplementiertes Verfahren zur Herstellung einer orthopädischen Versorgung sowie ein entsprechendes System zur Herstellung einer orthopädischen Versorgung.

Orthopädische Versorgungen umfassend Orthesen (korrektiv und präventiv) sowie Prothesen (Exo- und Endo-Prothesen). Prothesen sind heutzutage meist aus modularen Systemen aufgebaut. Dabei werden verschiedene Grundelemente (beispielsweise Prothesenschaft, Prothesenfuß/-hand und Prothesenohr) aufgebaut, welche über standardisierte Adapter zusammengeschraubt sind. Um den technischen Aufbau wird in der Regel eine kosmetische Versorgung erstellt, welche so gut wie möglich das natürliche Bein oder den Arm imitiert. Insofern besteht ein Bedürfnis darin, das Erscheinungsbild der Versorgung so gut wie möglich an die natürlichen Gegebenheiten anzupassen. Dennoch lässt sich der Unterschied zu einer natürlichen Extremität häufig nicht verbergen. Insofern gibt es Bestrebungen, solche Versorgungen an individuelle Bedürfnisse bzw. Präferenzen des Patienten bzw. Träger anzupassen.

Ein Beispiel einer entsprechenden orthopädischen Versorgung ist aus der DE 20 2017 000 442 U1 bekannt.

Auch Endo-Prothesen werden heute individuell an den Patienten von einem oder mehreren medizinischen Experten angepasst. Hierbei ist die ästhetische Anpassung naturgemäß weit weniger wichtig als die individualisierte Form und Funktion. Eine bemusterte Oberfläche ist hier eher funktional, da so z.B. das Einwachsverhalten verbessert werden kann.

Es ist bekannt, entsprechende orthopädische Versorgungen, insbesondere Orthesen computergestützt herzustellen. Insbesondere die abschließende Fertigung der Orthese wird in der Zwischenzeit zunehmend von computergestützten Verfahren begleitet (vgl. US 2014/0180185 A1). Es ist daher oft möglich, sehr individuelle Orthesen herzustellen, wobei die die Versorgung beschreibenden Versorgungsparameter persönliche Präferenzen sowie funktionelle Angaben enthalten. Die Generierung des Modells der orthopädischen Versorgung berücksichtigt aber im Stand der Technik häufig nur sehr wenige Informationen, die von dem Patienten und/oder dem die Fertigung der Orthese betreuenden Fachpersonal, z. B. dem Orthopädietechniker, Orthopäden oder Arzt, bereitgestellt werden. Es besteht daher ein Bedarf, entsprechende Orthesen weiter zu individualisieren und die Interaktion zwischen den beteiligten Personen und Systemen zu verbessern.

Selbiges gilt auch für präventive Orthesen (Präventhesen, Protektoren). Diese können aus medizinischen Gründen aber auch zum Schutz vor Verletzungen z.B. auch bei Kontakt-, Extrem- oder Motorsportarten getragen werden. Insb. eine ästhetische Anpassung (Individualisierung) kann bei nicht zwingend medizinisch notwendigen Versorgungen ein wichtiges Kriterium für die (Kauf-)Entscheidung sein. Diese präventiven Orthesen sollen den Patienten oder Träger in seiner Bewegung gezielt vor Verletzungen schützen oder vor externen Kräften oder Überbelastungen schützen. Dies kann beispielsweise eine individualisierte präventive Orthese nach einer Knieverletzung sein, die eine dosierte Belastung des Knies bei der Rehabilitation oder beim Sport erlaubt. Denkbar ist auch eine individualisierte Zervikalorthese als Protektor für Motorradfahrer oder Rennsportpiloten, welche diese vor Verletzungen bei Stürzen oder Unfällen schützt. Weitere Beispiele sind individualisierte Protektoren für Kontaktsportler wie Fußballspieler, Eishockeyspieler, American Football, Lacrosse u.v.m. sowohl im Amateur- aber insb. im Profi-Bereich.

Ausgehend von der US 2014/0180185 A1 ist es Aufgabe der vorliegenden Erfindung, ein verbessertes Verfahren zur Herstellung einer orthopädischen Versorgung anzugeben, bei dem Fehler bei der Herstellung der orthopädischen Versorgung reduziert werden. Insbesondere sollen Möglichkeiten geschaffen werden, um hochgradig individualisierte Versorgungen, vorzugsweise in einem iterativen Prozess, herzustellen. Weiterhin sollen die Funktion und die Akzeptanz entsprechender Versorgungen durch die Bereitstellung eines entsprechenden Verfahrens verbessert werden.

Die vorliegende Erfindung löst dieses Problem durch die Bereitstellung des computerimplementierten Verfahrens gemäß dem Anspruch 1.

Insbesondere wird die Aufgabe durch ein computerimplementiertes Verfahren zur Herstellung einer orthopädischen Versorgung gelöst, das die nachfolgenden Schritte umfasst:
a) Empfangen mindestens eines Datensatzes mit Patientendaten betreffend die Oberflächenstruktur eines Patienten;
b) Verarbeitung der Patientendaten zur Erstellung eines Patientenmodells unter Verwendung von in einer Datenbank gespeicherten Templates, wobei die Verarbeitung der Patientendaten ein Gewinnen eines Oberflächennetzes und von Knochendaten umfasst, und
   wobei die Erstellung des Patientenmodells eine Ausrichtungskorrektur in Bezug auf die Knochendaten zur Erzeugung eines korrigierten Oberflächennetzes umfasst,
   wobei das korrigierte Oberflächennetz unter Verwendung von Vektoren erzeugt wird, wobei die Vektoren Abstände oder Stützstellen innerhalb des Oberflächennetzes angeben und basierend auf den Templates gewonnen werden;
c) Nutzung des Patientenmodells zur Bestimmung von Patientenparametern;
d) Generierung einer (virtuellen) Darstellung der orthopädischen Versorgung unter Verwendung der Patientenparameter und von Versorgungsparametern;
e) Empfangen mindestens einer ersten Eingabe von einem Arzt oder Orthopädietechniker als einem ersten Benutzer und Empfangen mindestens einer zweiten Eingabe von einem Patienten als zweitem Benutzer;
f) Modifikation mindestens eines der Patientenparameter und/oder Versorgungsparameter basierend auf der Eingabe des ersten Benutzers und Modifizieren mindestens eines Versorgungsparameters basierend auf der Eingabe des zweiten Benutzers zur Erzeugung von modifizierten Patientenparametern und modifizierten Versorgungsparametern;
g) Generieren einer aktualisierten virtuellen Darstellung der orthopädischen Versorgung basierend auf den modifizierten Patientenparametern und den modifizierten Versorgungsparametern
h) Physische Erstellung der orthopädischen Versorgung unter Verwendung der modifizierten Versorgungsparameter und der modifizierten Patientenparameter.

Dabei umfassen die Patientenparameter Parameter, die einen Halsumfang, ein Gewicht eines Patienten, einen oder mehrere Winkel, eine Schulterbreite und/oder eine Position eines Adapters angeben.
Die Versorgungsparameter umfassen mindestens einen Konstruktionsparameter und/oder mindestens einen Funktionsparameter der orthopädischen Versorgung.

Ferner weist die Datenbank Authentifizierungsinformationen auf und das Verfahren umfasst ferner die folgenden Schritte, die unter Verwendung der Datenbank ausgeführt werden:
- ein Authentifizieren des ersten Benutzers vor dem Empfangen der Eingabe des ersten Benutzers; und
- ein Authentifizieren des zweiten Benutzers vor dem Empfangen der zweiten Eingabe des zweiten Benutzers; und
- ein Nachschlagen in der Datenbank, welche der Versorgungsparameter durch den zweiten Benutzer veränderbar sind, und
- ein Anzeigen nur der Versorgungsparameter zur Modifikation, die gemäß der Datenbank durch den zweiten Benutzer veränderbar sind.

Ein Gedanke der vorliegenden Erfindung besteht also darin, Rohdaten, wie sie beispielsweise ein Tomograph oder ein (optischer) Scanner liefert, zu nutzen, um ein Patientenmodell zu generieren.

Es kann also ein optisches Scanverfahren, z.B. unter Verwendung eines Laserscanners, eines laser-gestützten Scanners, oder eines stereoskopischen optischen Scanners mit Streifenlicht zur Gewinnung der Rohdaten durchgeführt werden. In einer Ausführungsform werden hierfür Kameras, insbesondere stereoskopische optische Kameras und/oder Kameras mit nur einem Linsensystem für digitale 3D-Rekonstruktion aus Bilderreihen eingesetzt. Nicht-optische Scanverfahren können ebenso zur Gewinnung der Rohdaten verwendet werden. Hierfür kann eine Computertomographie oder eine Magnetresonanztomographie durchgeführt werden.

Zusätzlich oder alternativ können die notwendigen Patientendaten auch manuell z.B. mittels eines Maßbands direkt am Patienten oder durch Vermessung eines Patientenabdrucks, z.B. eines Gipsnegativs, gewonnen werden. Auch taktile Vermessungsmöglichkeiten wie Messschieber, Lehren oder automatisierte taktile Messmaschinen sind denkbar.

Dieses Patientenmodell kann dann verwendet werden, um Patientenparameter abzuleiten, die letztendlich für die optimale Funktion der Versorgung notwendig sind.

Ein weiterer Gedanke der Erfindung besteht darin, Eingabe eines Benutzers, sei es eine Eingabe eines Orthopädietechnikers oder des Patienten, bei der Herstellung der Versorgung zu berücksichtigen. Entsprechende Eingaben können zur Verbesserung der Funktion sowie der Akzeptanz der Versorgung stark beitragen.

Das physische Erstellen der orthopädischen Versorgung kann ein Steuern mindestens einer Herstellungsmaschine, insbesondere eines 3D-Druckers umfassen. Allgemein sind auftragende Verfahren in diesem Bereich der Technik zu bevorzugen und führen zu Versorgungen, die bei geringem Gewicht äußerst stabil sind. Erfindungsgemäß können aber auch abtragende Verfahren, z.B. unter Verwendung einer CNC-Fräse eingesetzt werden. Auch kombinierte Verfahren bei denen unterschiedliche Drucker und/oder Herstellungsmaschinen verwendet werden sind denkbar.

Ein weiterer Gedanke der Erfindung besteht darin, den Herstellungsprozess zu optimieren. Dies setzt voraus, dass sowohl der die Herstellung betreuende Orthopädietechniker sowie der betroffene Patient möglichst intensiv in den Herstellungsprozess eingebunden ist. Daher schlägt die vorliegende Erfindung eine Visualisierung des Patientenmodells und/oder der Versorgung bzw. eines Modells der Versorgung vor.

Erfindungsgemäß können aber auch zusätzlich oder alternativ einzelne Patienten- und/oder Versorgungsparameter visualisiert werden. Hierfür können (einfache) Graphiken ggf. mit Beschriftungen verwendet werden. Beispielsweise können graphisch illustrierte Messblätter, wie sie in diesem Gebiet der Technik bekannt sind, angezeigt werden.

Die Visualisierung kann interaktiv oder statisch sein. Eine Visualisierung kann mittels einem 2D- oder einem 3D-Modell erfolgen. In einer Ausführungsform erfolgt ein Ausdrucken des Patienten- und/oder Versorgungsmodells, z.B. in 2D auf Papier oder 3D auf einem 3D-Drucker.

In einer Ausführungsform soll diese Visualisierung zu einem möglichst frühen Zeitpunkt stattfinden. In einer Ausführungsform wird eine virtuelle Darstellung des Patientenmodells generiert, wobei die orthopädische Versorgung zusammen mit dem Patientenmodell dargestellt wird. Eine entsprechende Darstellung kann unter Verwendung eines Webservers erfolgen. So lassen sich Bilder oder gar 3D-Daten mittels eines Webbrowsers visualisieren. In einer anderen Ausführungsform kann ein lokales Programm auf dem Computer des Nutzers (Orthopädietechniker oder Patienten) installiert sein, um eine geeignete Darstellung des Patientenmodells und/oder der Versorgung bereitzustellen.

In einer Ausführungsform kann die Visualisierung sowohl dem medizinischen Benutzer und/oder auch dem Patienten angezeigt werden. Für eine gesteigerte Akzeptanz der orthopädischen Versorgung ist es insbesondere hilfreich, wenn der Benutzer dem Patienten die Visualisierung der orthopädischen Versorgung in 3D z.B. in einem allgemein zugänglichen System wie z.B. einem Webbrowser oder in 2D z.B. in einem allgemein lesbaren Format wie z.B. einer PDF-Datei oder einem JPG-Bild zur Verfügung stellt.

Das Programm oder der Webbrowser können auch dafür genutzt werden, um Eingaben des oder der Benutzer(s) zu erfassen. In einer Ausführungsform wird die Darstellung der virtuellen Versorgung gegenüber dem Patientenmodell ausgerichtet. Dieser Ausrichtungsprozess kann automatisiert oder teilautomatisiert erfolgen. Der Nutzer des Verfahrens kann diese Darstellung verwenden, um notwendigen Anpassungen an dem Patientenmodell oder den Patientenparametern abzuleiten. Entsprechende Anpassungen können unmittelbar am Patientenmodell oder mittelbar an den Patientenparametern vorgenommen werden. Weiterhin können seitens des Benutzers Versorgungsparameter angepasst werden, wobei der Benutzer durch die virtuelle Darstellung in seiner Auswahl unterstützt wird.

Die Patientenparameter umfassen Parameter, die einen Halsumfang, ein Gewicht des Patienten, einen oder mehrere Winkel, z.B. einen Fußwinkel, eine Schulterbreite, aber auch eine Position eines Adapters, angeben. Ebenso umfassen die Versorgungsparameter mindestens einen Konstruktionsparameter und/oder Funktionsparameter. Die Versorgungsparameter können ferner mindestens einen Designparameter, z. B. Farbe der orthopädischen Versorgung, verwendetes Muster, umfassen. Der beschriebene Empfang mindestens einer Eingabe von mindestens einem Benutzer umfasst ein Empfangen mindestens einer ersten Eingabe von einem ersten Benutzer, nämlich von einem Arzt oder Orthopädietechniker, wobei in einer Ausführungsform das Patientenmodell und/oder mindestens ein Patientenparameter basierend auf der Eingabe des ersten Benutzers erfolgt. Das erfindungsgemäße Verfahren ermöglicht also eine Interaktion mit einem ersten Benutzer, insbesondere einem Orthopädietechniker.

Zusätzlich umfasst das Empfangen ein Empfangen mindestens einer zweiten Eingabe von mindestens einem zweiten Benutzer, nämlich einem Patienten, wobei ein Modifizieren mindestens eines Versorgungsparameters basierend auf der Eingabe des zweiten Benutzers erfolgt. Insbesondere in der Konstellation, in der der erste und der zweite Benutzer Eingaben tätigen, kann das erfindungsgemäße Verfahren die Synchronisation dieser Eingaben ermöglichen, so dass alle Änderungsvorschläge berücksichtigt werden.

Das Verfahren implementiert eine Authentifizierung des ersten und zweiten Benutzers, so dass keine unberechtigte oder ungewollte Veränderung der relevanten Parameter erfolgen kann. Es wird eine Berechtigungsdatenbank implementiert, die beispielsweise dem ersten Benutzer eine Vielzahl von Versorgungen zuordnet, die dieser bearbeiten darf. Beispielsweise kann diese Berechtigung darauf beruhen, dass der erste Benutzer die Versorgungen ursprünglich beauftragt hat. Die Berechtigungsdatenbank gibt an, welche Parameter, insbesondere welche Versorgungsparameter, durch den zweiten Benutzer veränderbar sind. Soweit es sich bei dem zweiten Benutzer um einen Patienten handelt, können hierdurch Einschränkungen vorgenommen werden, die den zweiten Benutzer davon abhalten, funktionell relevante Parameter zu verändern. Stattdessen kann es dem zweiten Benutzer ausschließlich ermöglicht werden, Versorgungsparameter, wie beispielsweise ein Erscheinungsbild der Versorgung eingeben, zu verändern.

In einer Ausführungsform kann die Berechtigungsdatenbank auch dafür zum Einsatz kommen, bestimmten Nutzern des gleichen Typs, z.B. eines Orthopädietechnikers oder Arztes, bestimmte Berechtigungslevel zuzuweisen. So könnte z.B. ein Orthopädietechniker der bereits mehrere orthopädische Versorgungen nach dem beschriebenen Verfahren durchgeführt hat oder im System als Experte markiert ist, Zugriff auf mehre Parameter hat (Expertenmodus).

In einer Ausführungsform umfassen die Patientendaten zusätzlich Kontaktdaten eines zweiten Benutzers bzw. des Patienten. Das Verfahren kann ein elektrisches Übermitteln einer Nachricht an den Patienten umfassen, die diesen auffordert, eine oder die bereits beschriebenen Eingaben zu tätigen. In einer Ausführungsform enthält die Nachricht eine URL, die es dem Benutzer ermöglicht, eine entsprechende Eingabemaske aufzurufen. Alternativ und zusätzlich können eine Benutzerkennung und/oder ein Passwort enthalten sein. Zusätzlich oder alternativ können die Patientendaten genutzt werden, um den Patienten zu authentifizieren. Vorzugsweise kann ein Patient nur dann Eingaben tätigen, wenn er sich erfolgreich authentifizieren konnte.

Die eingangs genannte Aufgabe wird des Weiteren durch einen computerlesbaren Speicher nach Anspruch 5 gelöst.

Es ergeben sich ähnliche Vorteile, wie diese bereits in Verbindung mit dem Verfahren beschrieben wurden.

Weiterhin wird die Aufgabe durch ein System zur Herstellung einer orthopädischen Versorgung nach Anspruch 6 gelöst, das zumindest einige der Schritte, die in Verbindung mit dem Verfahren beschrieben wurden implementiert.

In einer Ausführungsform handelt es sich bei dem System um ein System mit:
einem Entwurfsserver, der umfasst:
   - mindestens eine digitale Schnittstelle zum Empfangen eines Datensatzes mit Patientendaten;
   - mindestens eine Datenbank mit Trainingsdaten zur Erstellung eines Patientenmodells basierend auf den Patientendaten und den Trainingsdaten sowie mit Authentifizierungsinformationen;
   - mindestens eine Recheneinheit zur Generierung von 3D-Daten der orthopädischen Versorgung; und
   - einer Visualisierungseinrichtung, insbesondere einem Webserver, der dazu ausgebildet ist, die 3D-Daten in Form einer virtuellen Darstellung der Versorgung darzustellen und mindestens eine Eingabe mindestens eines Benutzers zu empfangen;
wobei die Recheneinheit, dazu geeignet ist, die folgenden Schritte durchzuführen:
   a) Empfangen des Datensatzes mit Patientendaten;
   b) Verarbeitung der Patientendaten zur Erstellung eines Patientenmodells unter Verwendung von in der Datenbank gespeicherten Templates, wobei die Verarbeitung der Patientendaten ein Gewinnen eines Oberflächennetzes und von Knochendaten umfasst, und
      wobei die Erstellung des Patientenmodells eine Ausrichtungskorrektur in Bezug auf die Knochendaten zur Erzeugung eines korrigierten Oberflächennetzes umfasst,
      wobei das korrigierte Oberflächennetz unter Verwendung von Vektoren erzeugt wird, wobei die Vektoren Abstände oder Stützstellen innerhalb des Oberflächennetzes angeben und basierend auf den Templates gewonnen werden;
   c) Nutzung des Patientenmodells zur Bestimmung von Patientenparametern;
   d) Generierung einer virtuellen Darstellung der orthopädischen Versorgung unter Verwendung der Patientenparameter und von Versorgungsparametern;
   e) Empfangen mindestens einer ersten Eingabe von einem Arzt oder Orthopädietechniker als einem ersten Benutzer und Empfangen mindestens einer zweiten Eingabe von einem Patienten als zweitem Benutzer;
   f) Modifizieren mindestens eines der Versorgungsparameter und/oder Patientenparameter basierend auf der Eingabe des ersten Benutzers und Modifizieren mindestens eines Versorgungsparameters basierend auf der Eingabe des zweiten Benutzers zur Erzeugung von modifizierten Patientenparametern und modifizierten Versorgungsparametern;
   g) Generieren einer aktualisierten virtuellen Darstellung der orthopädischen Versorgung basierend auf den modifizierten Patientenparametern und den modifizierten Versorgungsparametern;
   h) basierend auf den modifizierten Versorgungsparametern und den modifizierten Patientenparametern, Erzeugen von Herstellungsdaten zur physischen Erstellung der orthopädischen Versorgung.

Dabei umfassen die Patientenparameter Parameter, die einen Halsumfang, ein Gewicht eines Patienten, einen oder mehrere Winkel, eine Schulterbreite und/oder eine Position eines Adapters angeben.
Die Versorgungsparameter umfassen mindestens einen Konstruktionsparameter und/oder mindestens einen Funktionsparameter der orthopädischen Versorgung.

Dabei umfasst das Verfahren ferner die folgenden Schritte, die unter Verwendung der Datenbank ausgeführt werden:
- ein Authentifizieren des ersten Benutzers vor dem Empfangen der Eingabe des ersten Benutzers; und
- ein Authentifizieren des zweiten Benutzers vor dem Empfangen der zweiten Eingabe des zweiten Benutzers; und
- ein Nachschlagen in der Datenbank, welche der Versorgungsparameter durch den zweiten Benutzer veränderbar sind, und
- ein Anzeigen nur der Versorgungsparameter zur Modifikation, die gemäß der Datenbank durch den zweiten Benutzer veränderbar sind.

Auch bei dem System ergeben sich ähnliche oder identische Vorteile, wie diese bereits in Verbindung mit dem Verfahren beschrieben wurden.

Nachfolgend wird die Erfindung mittels mehrerer Ausführungsbeispiele beschrieben, die anhand von Abbildungen näher erläutert werden.

Hierbei zeigen:
- Fig. 1: einzelne Komponenten eines Systems zur Herstellung einer orthopädischen Versorgung;
- Fig. 2: Elemente des Herstellungsservers aus Fig. 1;
- Fig. 3: einzelne Verfahrensschritte zur Herstellung einer orthopädischen Versorgung;
- Fig. 4: Beispiel einer erfindungsgemäß gewonnenen Fußprothese; und
- Fig. 5: erfindungsgemäße Visualisierung der Fußprothese aus Fig. 4.

In der nachfolgenden Beschreibung werden für gleiche und gleichwirkende Teile dieselben Bezugsziffern verwendet.

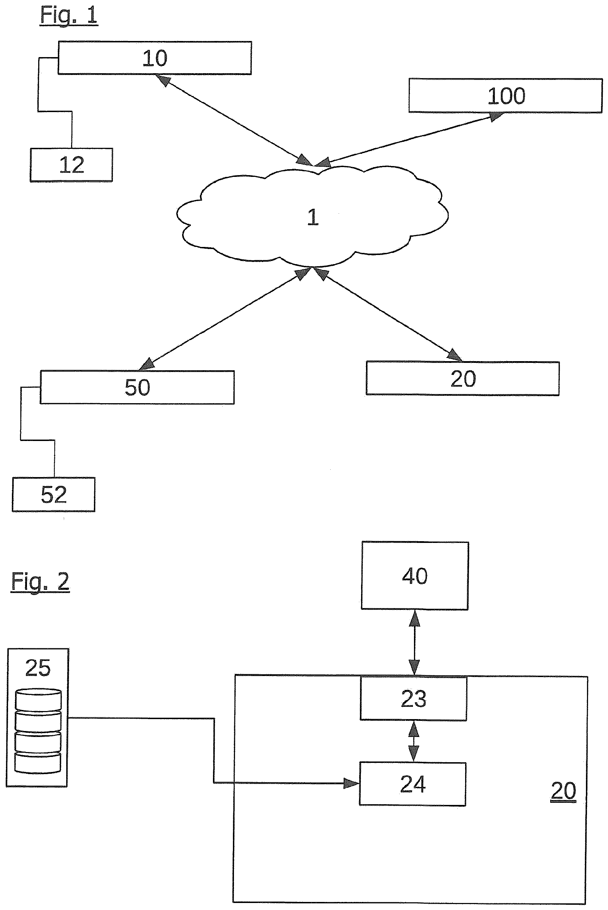

Fig. 1 zeigt einige der Komponenten, die im Zuge eines Ausführungsbeispiels des erfindungsgemäßen Herstellungsverfahrens miteinander kommunizieren. Hierbei handelt es sich um einen CPO-Computer 10, einen Patientencomputer 100, einen Herstellungsserver 50 sowie einen Entwurfsserver 20. All diese Komponenten sind kommunikativ über ein Netzwerk, in dem beschriebenen Ausführungsbeispiel über das Internet 1 miteinander verbunden.

Der CPO-Computer 10 umfasst einen optischen Scanner 12, zur Erfassung der Oberflächenstruktur eines Patienten. Hierdurch ergeben sich Rohdaten (ScanData).

Der Herstellungsserver 50 verfügt im beschriebenen Ausführungsbeispiel über einen 3D-Drucker 52 und kann somit eine beliebige Orthese herstellen, soweit ihm die hierfür notwendigen Daten von dem Entwurfsserver 20 bereitgestellt werden.

Die einzelnen Bestandteile des Entwurfsservers 20 werden näher anhand der Fig. 2 erläutert. Der Entwurfsserver 20 verfügt über eine Recheneinheit 24, die das nachfolgend beschriebene Verfahren zumindest teilweise implementiert. Des Weiteren ist eine Entwurfsschnittstelle 23 zur Kommunikation mit dem bereits beschriebenen CPO-Computer 10 und dem Patientencomputer 100 vorgesehen. In einem bevorzugten Ausführungsbeispiel erfolgt diese Kommunikation über einen Webserver 40, so dass die Computer 10, 100 keine eigene Software zur Kommunikation mit dem Entwurfsserver 20 benötigen. So kann auf die von dem Entwurfsserver 20 bereitgestellten Dienste mittels eines Webbrowsers zugegriffen werden.

Des Weiteren ist eine Datenbank 25 vorgesehen. Diese Datenbank 25 kann notwendige Modelldaten liefern, um seitens des Entwurfsservers 20 Modelle der Orthese herzustellen. Weiterhin können auf der Datenbank 25 Templates oder Parameter gespeichert sein, die es ermöglichen, ein individuelles Patientenmodell herzustellen. Die Datenbank 25 kann weiterhin Authentifizierungsinformationen enthalten, um Zugriffe auf den Entwurfsserver 20, insbesondere auf die von diesem angebotenen Dienste, zu verwalten.

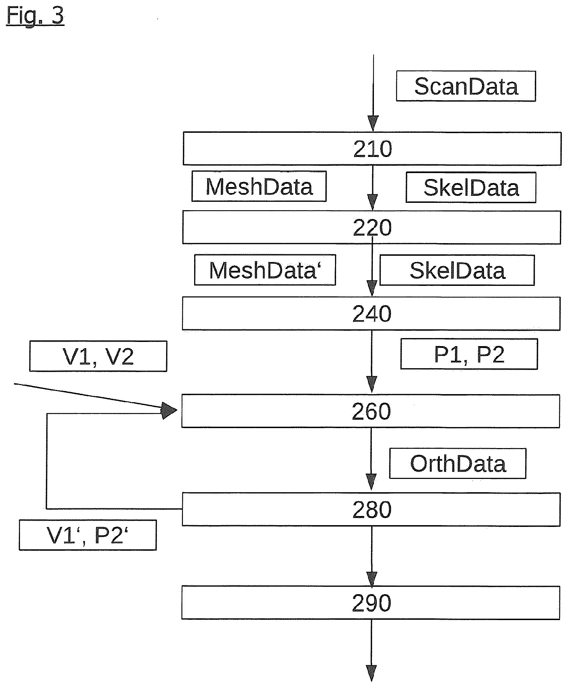

Wie anhand der Fig. 3 ersichtlich, empfängt der Entwurfsserver 20 die vom Orthopädietechniker mittels eines CPO-Computers 10 erfassten Rohdaten ScanData. Diese Rohdaten ScanData können beispielsweise im DICOM-Format bereitgestellt werden. In einem Vorverarbeitungsschritt 210 werden aus den Rohdaten ein Oberflächennetz des Patienten MeshData sowie Knochendaten SkelData gewonnen. Das Oberflächennetz MeshData - Mesh - kann als Dreiecksnetz, beispielsweise im STL Format erzeugt werden. Zur Gewinnung des Oberflächennetzes MeshData werden Oberflächenpunkte aus den Rohdaten ScanData extrahiert und die gewonnene Punktewolke in ein entsprechendes Netz eingebettet.

Auch bei den Knochendaten SkelData ist eine Modellierung als Dreiecksnetz erfindungsgemäß möglich. Bevorzugt werden aber Gelenke und Gelenkverbindungen, z.B. mittels Vektoren, modelliert und in einer geeigneten Datenstruktur erfasst. Weiterhin können zu jedem Gelenk übliche Freiheitsgrade im Hinblick auf Rotation- und/oder Translationsbewegungen gespeichert werden.

Das Oberflächennetz MeshData sowie die Knochendaten SkelData können in einem nachfolgenden Datenvalidierungs- und Optimierungsschritt 220 optimiert und validiert werden. In einem Ausführungsbeispiel erfolgt in dem Schritt 220 ein Anzeigen der Daten, wobei automatisch oder computergestützt Korrekturen vorgenommen werden. Basierend auf den vorgenommenen Korrekturen ergibt sich ein korrigiertes Oberflächennetz MeshData'. Die Korrektur kann ein Ausrichten der Knochendaten SkelData gemäß einer vorgegebenen, ggf. standardisierten Ausrichtung, umfassen, wobei das korrigierte Oberflächennetz MeshData' entsprechend der Ausrichtung der Knochendaten SkelData verformt wird.

Das korrigierte Oberflächennetz MeshData' sowie die Knochendaten SkelData werden in einem Patientenparameter-Extraktionsschritt 240 verarbeitet. Vorzugsweise wird in diesem Schritt 240 unter Zuhilfenahme der Datenbank 25 ein Patientenmodell gewonnen. Basierend auf diesem Patientenmodell werden Patientenparameter P1, P2 abgeleitet. Diese Patientenparameter P1, P2 können im Schritt der Orthesen-Modellerzeugung 260 verwendet werden, um ein Modell der Orthese zu erzeugen. Vorzugsweise liegen bereits einige Versorgungsparameter V1, V2 vor, die Parameter der Orthese angeben. Das Orthesenmodell und ggf. auch das Patientenmodell können in einem Visualisierungsschritt 220 visualisiert werden.

In einem Ausführungsbeispiel kann die Visualisierung genutzt werden, um einige der Parameter, beispielsweise den Versorgungsparameter V1 und den Patientenparameter P2 anzupassen. Eine entsprechende Anpassung kann durch den Orthopädietechniker oder ggf. durch den Patienten erfolgen. Die Anpassung kann in einem Schritt oder in getrennten Schritten vorgenommen werden. Nach einer Veränderung der Parameter kann in einer erneuten Orthesen-Modellerzeugung 260 ein aktualisiertes Modell der Orthese, z.B. unter Verwendung des modifizierten Versorgungsparameters V1' und des modifizierten Patientenparameters P2', erzeugt werden. Es ergeben sich Orthesen-Modelldaten OrthData, die soweit sie nach einer erneuten Visualisierung 280 die Zustimmung der Benutzer finden, dem Herstellungsserver 50 zugeführt werden, um eine Orthesen-Fertigung 290 einzuleiten.

Datenvalidierungs- und Optimierungsschritt 220 umfasst in einem Ausführungsbeispiel eine Ausrichtungskorrektur, z.B. gemäß einer bestimmten standardisierten Vorgabe.

Um die Haltung eines Scans zu korrigieren bedarf es des Oberflächennetzes MeshData und der Knochendaten SkelData. Knochendaten SkelData können, wie erläutert, ein vereinfachtes Knochengerüst sein, welches im Inneren des erfassten Objekts und somit innerhalb des Oberflächennetzes MeshData liegt. In einem Ausführungsbeispiel liegen Daten vor, die die Interaktion zwischen den Knochendaten SkelData und dem Oberflächennetz MeshData modellieren. Beispielsweise können Vektoren Abstände oder Stützstellen innerhalb des Oberflächennetzes angeben. Entsprechende Vektoren können basieren auf Templates, die in der Datenbank 25 gespeichert sind, gewonnen werden.

Eine Bewegung der Knochen für die Ausrichtungskorrektur führt zu einer Verformung des modellierten 3D Objektes und somit zu einem veränderten Oberflächennetz.

Erfindungsgemäß können die anatomischen Gegebenheiten berücksichtigt werden. In einem Ausführungsbeispiel werden die Knochendaten SkelData unter Verwendung der Templates an das gewonnene Oberflächennetz angepasst, und durch weitere Prozessschritte verbessert, um eine möglichst realistische Verformung modellieren zu können. Das sich ergebende korrigierte Oberflächennetz MashData' kann zur Extraktion von Patientenparametern genutzt werden.

Z.B. bei der Herstellung einer patientenindividuellen Knöchelorthese (Ankle-Foot-Orthosis, AFO) kann ein Unterschenkelscan betrachtet werden. Der Scan bzw. die zugehörigen Rohdaten ScanData können erfindungsgemäß im Schritt 220 in eine korrigierte Haltung gebracht werden. Dazu wird in einem ersten Schritt die Orientierung des Fußes identifiziert und in eine definierte Orientierung gebracht.

Um die Haltung beim Scan beurteilen zu können, werden Winkel des Knochenmodelles - Knochendaten SkelData - zusammen mit weiteren biomechanischen Achsen und Ebenen untersucht. Weichen diese Winkel von einem gewählten Maß ab, werden die Knochendaten SkelData angepasst - ausgerichtet, wodurch sich auch der Scan ändert (korrigiertes Oberflächennetz MeshData).

Erfindungsgemäß können beispielsweise folgende Schritte durchgeführt werden:
- Erstellen eines Patientenmodells basierend auf den Knochendaten SkelData und den Oberflächendaten MeshData;
- Finden des Fußes in dem Patientenmodell;
- Nutzung der vorhandenen Daten, um eine Auflageebene zu bestimmen;
- Modifizieren eines auf den Knochendaten SkelData basierenden Modelles bis die Auflageebene parallel zu einer virtuellen Bodenfläche ausgerichtet ist, z.B. Rotation um das Fußgelenk (erste Ausrichtungskorrektur);
- Modifizieren des Modells bis der Winkel eines Kniegelenks einen vorgegebenen Wert einnimmt (zweite Ausrichtungskorrektur);

Modifizieren der Oberflächendaten MeshData basierend auf der ersten und zweiten Ausrichtungskorrektur zum Erhalt der korrigierten Oberflächendaten MeshData'.

Die beschriebene Ausrichtungskorrektur kann eine Extraktion von Patientendaten erst ermöglichen oder das Ergebnis deutlich verbessern.

Der Patientenparameter-Extraktionsschritt 240 folgt in einem Ausführungsbeispiel dem nachfolgenden Schema.

Für die Konstruktion einer patientenindividuellen Knöchelorthese (Ankle-Foot-Orthosis, AFO) bedarf es beispielsweise verschiedener Längen- und Umfangsmaße des Fußes und Unterschenkels (Patientenparameter). Um diese aus den Rohdaten ScanData zu extrahieren kann wie folgt vorgegangen werden:
Die Oberflächendaten MeshData des Patienten werden ausgerichtet, und in ein Referenzsystem gebracht, aus dem sich schließen lässt, welcher Teil des Scans den Fuß, und welcher das Bein darstellt. In den Oberflächendaten MeshData wird dann ein vereinfachtes Fußmodell platziert.

Dieses Fußmodell, das ggf. in der Datenbank 25 gespeichert ist, ist bekannt und lässt sich anhand seiner Freiheitsgrade - bspw. Länge, Skalierung, Rotation von Subteilen, etc. - verändern.

Die Freiheitsgrade des Fußmodells werden in einem Optimierungsverfahren an Oberflächendaten MeshData angepasst bis die Korrelation von MeshDaten und Fußmodell optimal ist (möglichst geringe Abweichung). In einem Ausführungsbeispiel wird entsprechend mit dem "Significant Points" Modell (SPM), welches zur Abnahme der Maße verwendet wird, verfahren.

Das SPM besteht aus Punkten und Ebenen, zwischen denen Maße abgenommen werden. Von dem SPM werden die Maßabnahmepunkte auf die Oberflächendaten MeshData oder die korrigierten Oberflächendaten MeshData' projiziert. Dies geschieht je nach Messart unterschiedlich. Umfangsmaße benötigen eine Schnittebene, Längenmaße lediglich Punkte.

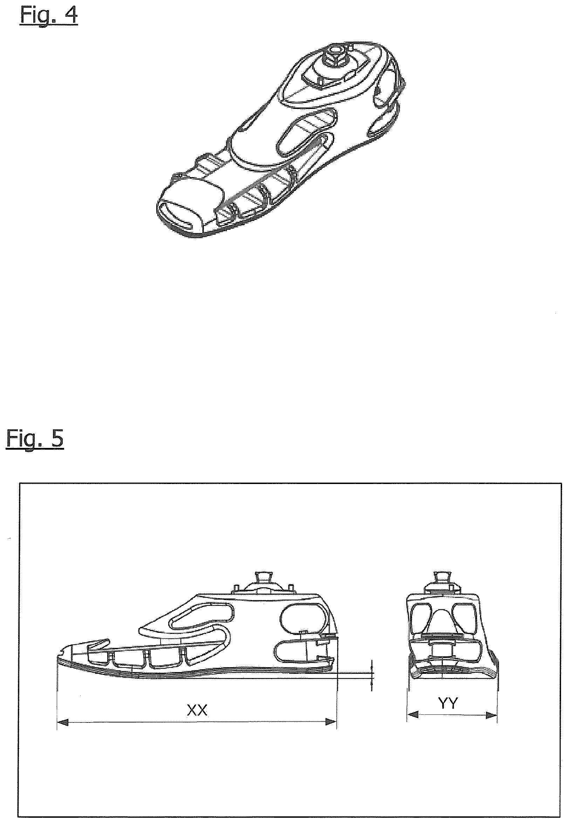

Zwischen diesen projizierten Punkten, oder entlang der Schnittebenen werden die Maße abgenommen. Diese können zum Beispiel in ein Maßtblatt eingetragen werden. In einem Ausführungsbeispiel umfasst der Visualisierungsschritt 220 das Erzeugen eines 3D-Bilds der Orthese. In einem anderen Ausführungsbeispiel wird zur Visualisierung von Patientendaten ein Maßblatt angezeigt und/oder ausgedruckt, dass ähnlich oder identisch aussieht wie dies in der Fig. 5 gezeigt ist.

Vorhergehend wurden ein Herstellungsverfahren sowie ein System zur Herstellung einer Orthese beschrieben. Mit den erfindungswesentlichen Merkmalen lässt sich auch ohne Weiteres eine Prothese, z.B. eine Fußprothese, wie diese in Fig. 4 gezeigt ist herstellen.

Auch die Herstellung von Endo-Prothesen oder präventiven Orthesen (Protektoren für Rehabilitation und Sport) nach demselben Verfahren ist denkbar.

### Bezugszeichenliste:

- 1: Internet
- 10: CPO-Computer
- 12: Scanner
- 20: Entwurfsserver
- 23: Entwurfsserverschnittstelle
- 24: Recheneinheit
- 25: Datenbank
- 40: Webserver
- 50: Herstellungsserver
- 52: 3D-Druck
- 100: Patienten-Computer
- 210: Vorverarbeitung (z.B. Knochendatenextraktion)
- 220: Datenvalidierung und Optimierung
- 240: Patientenparameter-Extraktion
- 260: Orthesen-Modellerzeugung
- 280: Visualisierung
- 290: Orthesen-Fertigung
- ScanData: Rohdaten
- MeshData: Oberflächennetz
- MeshData': Korrigiertes Oberflächennetz
- OrthData: 3D Orthesen-Modelldaten
- SkelData: Knochendaten
- P1, P2, P2': Patientenparameter
- V1, V2, V1': Orthesenparameter

## Patentansprüche

1. Computerimplementiertes Verfahren zur Herstellung einer orthopädischen Versorgung, umfassend die Schritte:
a. Empfangen mindestens eines Datensatzes mit Patientendaten (ScanData) betreffend die Oberflächenstruktur eines Patienten;
b. Verarbeitung der Patientendaten (ScanData) zur Erstellung eines Patientenmodells unter Verwendung von in einer Datenbank (25) gespeicherten Templates,
wobei die Verarbeitung der Patientendaten (ScanData) ein Gewinnen eines Oberflächennetzes (MeshData) und von Knochendaten (SkelData) umfasst, und
wobei die Erstellung des Patientenmodells eine Ausrichtungskorrektur in Bezug auf die Knochendaten (SkelData) zur Erzeugung eines korrigierten Oberflächennetzes (MeshData') umfasst,
wobei das korrigierte Oberflächennetz (MeshData') unter Verwendung von Vektoren erzeugt wird, wobei die Vektoren Abstände oder Stützstellen innerhalb des Oberflächennetzes (MeshData) angeben und basierend auf den Templates gewonnen werden;
c. Nutzung des Patientenmodells zur Bestimmung von Patientenparametern (P1, P2);
d. Generierung einer virtuellen Darstellung der orthopädischen Versorgung unter Verwendung der Patientenparameter (P1, P2) und von Versorgungsparametern (V1, V2);
e. Empfangen mindestens einer ersten Eingabe von einem Arzt oder Orthopädietechniker als einem ersten Benutzer und Empfangen mindestens einer zweiten Eingabe von einem Patienten als zweitem Benutzer;
f. Modifikation mindestens eines der Patientenparameter (P1, P2) und/oder Versorgungsparameter (V1, V2) basierend auf der Eingabe des ersten Benutzers und Modifizieren mindestens eines Versorgungsparameters (V1, V2) basierend auf der Eingabe des zweiten Benutzers zur Erzeugung von modifizierten Patientenparametern (P2') und modifizierten Versorgungsparametern (V1');
g. Generieren einer aktualisierten virtuellen Darstellung der orthopädischen Versorgung basierend auf den modifizierten Patientenparametern (P2') und den modifizierten Versorgungsparametern (V1');
h. Physische Erstellung der orthopädischen Versorgung unter Verwendung der modifizierten Versorgungsparameter (V1') und der modifizierten Patientenparameter (P2'),
wobei die Patientenparameter (P1, P2) Parameter umfassen, die einen Halsumfang, ein Gewicht eines Patienten, einen oder mehrere Winkel, eine Schulterbreite und/oder eine Position eines Adapters angeben und
wobei die Versorgungsparameter (V1, V2) mindestens einen Konstruktionsparameter und/oder mindestens einen Funktionsparameter der orthopädischen Versorgung umfassen,
wobei die Datenbank (25) ferner Authentifizierungsinformationen aufweist und das Verfahren ferner die folgenden Schritte umfasst, die unter Verwendung der Datenbank (25) ausgeführt werden:
- ein Authentifizieren des ersten Benutzers vor dem Empfangen der Eingabe des ersten Benutzers; und
- ein Authentifizieren des zweiten Benutzers vor dem Empfangen der zweiten Eingabe des zweiten Benutzers; und
- ein Nachschlagen in der Datenbank (25), welche der Versorgungsparameter (V1, V2) durch den zweiten Benutzer veränderbar sind, und
- ein Anzeigen nur der Versorgungsparameter (V1, V2) zur Modifikation, die gemäß der Datenbank (25) durch den zweiten Benutzer veränderbar sind.

2. Verfahren nach Anspruch 1,
**dadurch gekennzeichnet, dass**
der Schritt h) ein Steuern mindestens einer Herstellungsmaschine (50), nämlich mindestens eines 3D Druckers, umfasst.

3. Verfahren nach einem der vorhergehenden Ansprüche,
**gekennzeichnet durch**
einen Schritt d1), in dem eine virtuelle Darstellung des Patientenmodells generiert wird, wobei in Schritt d) die virtuelle Darstellung der orthopädischen Versorgung zusammen mit der virtuellen Darstellung des Patientenmodells unter Verwendung eines Webservers, dargestellt wird.

4. Verfahren nach einem der vorhergehenden Ansprüche, **dadurch gekennzeichnet, dass**
die Patientendaten (ScanData) Kontaktdaten eines Patienten umfassen, wobei das Verfahren aufweist:
i. elektronisches Übermitteln einer Nachricht mit einer URI an den Patienten unter Verwendung der Kontaktdaten,
ii. Authentifizieren des Patienten unter Verwendung der Kontaktdaten derart, dass der Patient als zweiter Benutzer die mindestens eine zweite Eingabe vornehmen kann.

5. Computerlesbarer Speicher mit Instruktionen, die bei der Ausführung auf mindestens einer Recheneinheit diese dazu veranlassen, das Verfahren gemäß einem der vorhergehenden Ansprüche auszuführen.

6. System zur Herstellung einer orthopädischen Versorgung, wobei das Verfahren gemäß einem der Ansprüche 1 bis 4 implementiert wird, mit:
einem Entwurfsserver (20), der umfasst:
- mindestens eine digitale Schnittstelle (23) zum Empfangen eines Datensatzes mit Patientendaten (ScanData);
- mindestens eine Datenbank (25) mit Trainingsdaten zur Erstellung eines Patientenmodells basierend auf den Patientendaten (ScanData) und den Trainingsdaten sowie mit Authentifizierungsinformationen;
- mindestens eine Recheneinheit zur Generierung von 3D-Daten der orthopädischen Versorgung; und
- eine Visualisierungseinrichtung, die dazu ausgebildet ist, die 3D-Daten in Form einer virtuellen Darstellung der Versorgung darzustellen und mindestens eine Eingabe mindestens eines Benutzers zu empfangen;
wobei die Recheneinheit dazu geeignet ist, die folgenden Schritte durchzuführen:
a) Empfangen des Datensatzes mit Patientendaten (ScanData);
b) Verarbeitung der Patientendaten (ScanData) zur Erstellung eines Patientenmodells unter Verwendung von in der Datenbank (25) gespeicherten Templates,
wobei die Verarbeitung der Patientendaten (ScanData) ein Gewinnen eines Oberflächennetzes (MeshData) und von Knochendaten (SkelData) umfasst, und
wobei die Erstellung des Patientenmodells eine Ausrichtungskorrektur in Bezug auf die Knochendaten (SkelData) zur Erzeugung eines korrigierten Oberflächennetzes (MeshData') umfasst,
wobei das korrigierte Oberflächennetz (MeshData') unter Verwendung von Vektoren erzeugt wird, wobei die Vektoren Abstände oder Stützstellen innerhalb des Oberflächennetzes (MeshData) angeben und basierend auf den Templates gewonnen werden;
c) Nutzung des Patientenmodells zur Bestimmung von Patientenparametern (P1, P2);
d) Generierung einer virtuellen Darstellung der orthopädischen Versorgung unter Verwendung der Patientenparameter (P1, P2) und von Versorgungsparametern (V1, V2);
e) Empfangen mindestens einer ersten Eingabe von einem Arzt oder Orthopädietechniker als einem ersten Benutzer und Empfangen mindestens einer zweiten Eingabe von einem Patienten als zweitem Benutzer;
f) Modifizieren mindestens eines der Versorgungsparameter (V1, V2) und/oder Patientenparameter (P1, P2) basierend auf der Eingabe des ersten Benutzers und Modifizieren mindestens eines Versorgungsparameters (V1, V2) basierend auf der Eingabe des zweiten Benutzers zur Erzeugung von modifizierten Patientenparametern (P2') und modifizierten Versorgungsparametern (V1');
g) Generieren einer aktualisierten virtuellen Darstellung der orthopädischen Versorgung basierend auf den modifizierten Patientenparametern (P2') und den modifizierten Versorgungsparametern (V1');
h) basierend auf den modifizierten Versorgungsparametern (V1') und den modifizierten Patientenparametern (P2'), Erzeugen von Herstellungsdaten zur physischen Erstellung der orthopädischen Versorgung
wobei die Patientenparameter (P1, P2) Parameter umfassen, die einen Halsumfang, ein Gewicht eines Patienten, einen oder mehrere Winkel, eine Schulterbreite und/oder eine Position eines Adapters angeben und
wobei die Versorgungsparameter (V1, V2) mindestens einen Konstruktionsparameter und/oder mindestens einen Funktionsparameter der orthopädischen Versorgung umfassen,
wobei das Verfahren ferner die folgenden Schritte umfasst, die unter Verwendung der Datenbank (25) ausgeführt werden:
- ein Authentifizieren des ersten Benutzers vor dem Empfangen der Eingabe des ersten Benutzers; und
- ein Authentifizieren des zweiten Benutzers vor dem Empfangen der zweiten Eingabe des zweiten Benutzers; und
- ein Nachschlagen in der Datenbank (25), welche der Versorgungsparameter (V1, V2) durch den zweiten Benutzer veränderbar sind, und
- ein Anzeigen nur der Versorgungsparameter (V1, V2) zur Modifikation, die gemäß der Datenbank (25) durch den zweiten Benutzer veränderbar sind.

## Claims

1. A computer-implemented method of manufacturing an orthopedic device, comprising the following steps:
a. Receiving at least one data set with patient data (ScanData) related to the surface structure of a patient;
b. Processing the patient data (ScanData) to create a patient model using templates stored in a database (25),
wherein said processing of the patient data (ScanData) comprises obtaining a surface mesh (MeshData) and bone data (SkelData), and wherein said creating of said patient model comprises an alignment correction related to the bone data (SkelData) for creating a corrected surface mesh (MeshData'),
wherein the corrected surface mesh (MeshData') is created using vectors, the vectors indicating distances or support points within the surface mesh (MeshData) and being obtained based on said templates;
c. Using of the patient model to determine patient parameters (P1, P2);
d. Generation of a virtual representation of the orthopedic device using patient parameters (P1, P2) and device parameters (V1, V2);
e. Receiving at least a first input from a physician or prosthetist as a first user and receiving at least a second input from a patient as a second user;
f. Modifying at least one of the patient parameters (P1, P2) and/or device parameters (V1, V2) based on the input of the first user and modifying at least one device parameter (V1, V2) based on the input of the second user to generate modified patient parameters (P2') and modified device parameters (V1');
g. Generating an updated virtual representation of the orthopedic device based on the modified patient parameters (P2') and the modified device parameters (V1');
h. Physical creation of the orthopedic device using the modified device parameters (V1') and the modified patient parameters (P2'),
wherein the patient parameters (P1, P2) comprise parameters indicating a neck circumference, a weight of a patient, one or more angles, a shoulder width and/or a position of an adapter, and
wherein the device parameters (V1, V2) comprise at least one design parameter and/or at least one functional parameter of the orthopedic device,
wherein the database (25) further comprises authentication information, and the method further comprises the following steps performed using the database (25):
- authenticating the first user prior to receiving the first user's input; and
- authenticating the second user prior to receiving the second user's second input; and
- looking up in the database (25) which of the device parameters (V1, V2) are modifiable by the second user, and
- displaying for modification only the device parameters (V1, V2) that are modifiable by the second user according to the database (25).

2. The method according to claim 1,
**characterized in that**
step h) comprises controlling at least one manufacturing machine (50), namely at least one 3D printer.

3. A method according to any one of the preceding claims,
**characterized by**
a step d1) of generating a virtual representation of the patient model, wherein in step d) the orthopedic device is displayed together with the virtual representation of the patient model using a web server.

4. A method according to any one of the preceding claims,
**characterized by**
the patient data (ScanData) comprising contact data of a patient,
the method comprising:
i. electronic transmission of a message with a URI to the patient using the contact details,
ii. Authenticating the patient using the contact information in such a way that the patient can make the at least one second input as a second user.

5. A computer-readable medium containing instructions that, when executed on at least one computing unit, cause the computing unit to execute the method according to any one of the preceding claims.

6. A system for manufacturing an orthopedic device, wherein the method is implemented according to any one of the claims 1 to 4, comprising:
a design server (20) comprising:
- at least one digital interface (23) for receiving a data set with patient data (ScanData);
- at least one database (25) containing training data for creating a patient model based on the patient data (ScanData) and the training data, and authentication information;
- at least one computing unit for generating 3D data of the orthopedic device; and
- a visualization device designed to display the 3D data in the form of a virtual representation of the orthosis and to receive at least one input from at least one user;
wherein the computing unit is adapted to perform the following steps:
a) Receiving the data set with patient data (ScanData);
b) processing the patient data (ScanData) to create a patient model using templates stored in the database (25),
wherein said processing of the patient data (ScanData) comprises obtaining a surface mesh (MeshData) and bone data (SkelData), and wherein said creating of said patient model comprises an alignment correction related to the bone data (SkelData) for creating a corrected surface mesh (MeshData'),
wherein the corrected surface mesh (MeshData') is created using vectors, the vectors indicating distances or support points within the surface mesh (MeshData) and being obtained based on said templates;
c) Use of the patient model to determine patient parameters (P1, P2);
d) Generation of a virtual representation of the orthopedic device using patient parameters (P1, P2) and device parameters (V1, V2);
e) Receiving at least a first input from a physician or prosthetist as a first user and receiving at least a second input from a patient as a second user;
f) Modifying at least one of the device parameters (V1, V2) and/or patient parameters (P1, P2) based on the input of the first user, and modifying at least one of the device parameters (V1, V2) based on the input of the second user to generate modified patient parameters (P2') and modified device parameters (V1');
g) Generating an updated virtual representation of the orthopedic device based on the modified patient parameters (P2') and the modified device parameters (V1');
h) Based on the modified device parameters (V1') and the modified patient parameters (P2'), generating manufacturing data for the physical creation of the orthopedic device.
wherein the patient parameters (P1, P2) comprise parameters indicating a neck circumference, a weight of a patient, one or more angles, a shoulder width and/or a position of an adapter, and
wherein the device parameters (V1, V2) comprise at least one design parameter and/or at least one functional parameter of the orthopedic device
wherein the method further comprises the following steps performed using the database (25):
- authenticating the first user prior to receiving the first user's input; and
- authenticating the second user prior to receiving the second user's second input; and
- looking up in the database (25) which of the device parameters (V1, V2) are modifiable by the second user, and
- displaying for modification only those device parameters (V1, V2) that are modifiable by the second user according to the database (25).

## Revendications

1. Procédé mis en œuvre par ordinateur pour la réalisation d'un produit orthopédique, comprenant les étapes suivantes :
a. réception d'au moins un jeu de données comportant des données de patient (ScanData) concernant la structure de surface d'un patient ;
b. traitement des données du patient (ScanData) pour élaborer un modèle de patient à l'aide de modèles enregistrés dans une base de données (25),
ledit traitement des données du patient (ScanData) comprenant l'obtention d'un maillage de surface (MeshData) et de données osseuses (SkelData), et
ladite élaboration du modèle de patient comprenant une correction de l'alignement en fonction des données osseuses (SkelData) pour produire un maillage de surface corrigé (MeshData'),
ledit maillage de surface corrigé (MeshData') étant produit à l'aide de vecteurs, lesquels vecteurs sont indicateurs de distances ou de points d'appui au sein du maillage de surface (MeshData) et étant obtenus à partir desdits modèles ;
c. exploitation du modèle du patient pour déterminer des paramètres du patient (P1, P2) ;
d. génération d'une représentation virtuelle du produit orthopédique à l'aide des paramètres du patient (P1, P2) et de paramètres du produit (V1, V2) ;
e. réception d'au moins une première entrée réalisée par un médecin ou technicien orthopédiste à titre de premier utilisateur, et réception d'au moins une deuxième entrée réalisée par un patient à titre de deuxième utilisateur ;
f. modification d'au moins un des paramètres du patient (P1, P2) et/ou paramètres du produit (V1, V2) compte tenu de l'entrée réalisée par le premier utilisateur, et modification d'au moins un paramètre du produit (V1, V2) compte tenu de l'entrée réalisée par le deuxième utilisateur pour produire des paramètres du patient modifiés (P2') et des paramètres du produit modifiés (V1') ;
g. génération d'une représentation virtuelle actualisée du produit orthopédique compte tenu des paramètres du patient modifiés (P2') et des paramètres du produit modifiés (V1') ;
h. élaboration physique du produit orthopédique au moyen des paramètres du produit modifiés (V1') et des paramètres du patient modifiés (P2') ;
lesdits paramètres du patient (P1, P2) comprenant des paramètres indicateurs du tour de cou, du poids du patient, d'un ou plusieurs angles, de la largeur d'épaules et/ou de la position d'un adaptateur, et
lesdits paramètres du produit (V1, V2) comprenant au moins un paramètre de construction et/ou au moins un paramètre fonctionnel concernant le produit orthopédique,
ladite base de données (25) présentant en outre des informations d'authentification et le procédé comprenant par ailleurs les étapes suivantes qui sont exécutées à l'aide de ladite base de données (25) :
- une authentification du premier utilisateur avant la réception de l'entrée réalisée par le premier utilisateur, et
- une authentification du deuxième utilisateur avant la réception de la deuxième entrée réalisée par le deuxième utilisateur, et
- une consultation de la base de données (25) pour connaître ceux des paramètres du produit (V1, V2) qui peuvent être modifiés par le deuxième utilisateur, et
- un affichage uniquement de ceux des paramètres du produit (V1, V2) à modifier qui, selon la base de données (25), peuvent être modifiés par le deuxième utilisateur.

2. Procédé selon la revendication 1,
**caractérisé en ce que**
l'étape h) comprend la commande d'au moins une machine de fabrication (50), à savoir au moins une imprimante 3D.

3. Procédé selon l'une des revendications précédentes,
**caractérisé par**
une étape d1) au cours de laquelle une représentation virtuelle du modèle du patient est générée, étant entendu qu'à l'étape d) la représentation virtuelle du produit orthopédique et la représentation virtuelle du modèle du patient sont représentées conjointement, au moyen d'un serveur Web.

4. Procédé selon l'une des revendications précédentes,
**caractérisé en ce que**
les données du patient (ScanData) comprennent des données de contact du patient, le procédé comprenant :
i. la transmission électronique d'un message comportant un identifiant URI auprès du patient, au moyen des données de contact,
ii. l'authentification du patient au moyen des données de contact de manière que le patient puisse, à titre de deuxième utilisateur, réaliser au moins une deuxième entrée.

5. Mémoire lisible par ordinateur, dotée d'instructions qui, lors de leur exécution sur au moins une unité informatique, amènent celle-ci à mettre en œuvre le procédé selon l'une des revendications précédentes.

6. Système de réalisation de produit orthopédique, dans lequel est mis en œuvre le procédé selon l'une des revendications 1 à 4 et comprenant :
un serveur de conception (20), comprenant :
- au moins une interface numérique (23) destinée à recevoir un jeu de données comportant des données de patient (ScanData) ;
- au moins une base de données (25) comportant des données d'entraînement permettant d'élaborer un modèle du patient à partir des données du patient (ScanData) et des données d'entraînement, et comportant également des informations d'authentification ;
- au moins une unité informatique permettant de générer des données 3D du produit orthopédique ; et
- un dispositif de visualisation conçu pour représenter les données 3D sous la forme d'une représentation virtuelle du produit et pour recevoir au moins une entrée d'au moins un utilisateur ;
ladite unité informatique étant apte à exécuter les étapes suivantes :
a) réception du jeu de données comportant les données du patient (ScanData) ;
b) traitement des données du patient (ScanData) pour élaborer un modèle de patient à l'aide de modèles enregistrés dans la base de données (25),
ledit traitement des données du patient (ScanData) comprenant l'obtention d'un maillage de surface (MeshData) et de données osseuses (SkelData), et
ladite élaboration du modèle de patient comprenant une correction de l'alignement en fonction des données osseuses (SkelData) pour produire un maillage de surface corrigé (MeshData'),
ledit maillage de surface corrigé (MeshData') étant produit à l'aide de vecteurs, lesquels vecteurs sont indicateurs de distances ou de points d'appui au sein du maillage de surface (MeshData) et obtenus à partir desdits modèles ;
c) exploitation du modèle du patient pour déterminer des paramètres du patient (P1, P2) ;
d) génération d'une représentation virtuelle du produit orthopédique à l'aide des paramètres du patient (P1, P2) et de paramètres du produit (V1, V2) ;
e) réception d'au moins une première entrée réalisée par un médecin ou technicien orthopédiste à titre de premier utilisateur, et réception d'au moins une deuxième entrée réalisée par un patient à titre de deuxième utilisateur ;
f) modification d'au moins un des paramètres du produit (V1, V2) et/ou paramètres du patient (P1, P2) compte tenu de l'entrée réalisée par le premier utilisateur, et modification d'au moins un paramètre du produit (V1, V2) compte tenu de l'entrée réalisée par le deuxième utilisateur pour produire des paramètres du patient modifiés (P2') et des paramètres du produit modifiés (V1') ;
g) génération d'une représentation virtuelle actualisée du produit orthopédique compte tenu des paramètres du patient modifiés (P2') et des paramètres du produit modifiés (V1') ;
h) compte tenu des paramètres du produit modifiés (V1') et des paramètres du patients modifiés (P2'), production de données de fabrication concernant l'élaboration physique du produit orthopédique,
lesdits paramètres du patient (P1, P2) comprenant des paramètres indicateurs du tour de cou, du poids du patient, d'un ou plusieurs angles, de la largeur d'épaules et/ou de la position d'un adaptateur, et
lesdits paramètres du produit (V1, V2) comprenant au moins un paramètre de construction et/ou au moins un paramètre fonctionnel concernant le produit orthopédique,
le procédé comprenant en outre les étapes suivantes, exécutées à l'aide de ladite base de données (25) :
- une authentification du premier utilisateur avant la réception de l'entrée réalisée par le premier utilisateur, et
- une authentification du deuxième utilisateur avant la réception de la deuxième entrée réalisée par le deuxième utilisateur, et
- une consultation de la base de données (25) pour connaître ceux des paramètres du produit (V1, V2) qui peuvent être modifiés par le deuxième utilisateur, et
- un affichage uniquement de ceux des paramètres du produit (V1, V2) à modifier qui, selon la base de données (25), peuvent être modifiés par le deuxième utilisateur.
